# EUROPÄISCHE PATENTSCHRIFT

(11) **EP 1 074 214 B1**
(45) Veröffentlichungstag und Bekanntmachung des Hinweises auf die Patenterteilung: **02.07.2003**
(21) Anmeldenummer: 00110680.6
(22) Anmeldetag: 19.05.2000
(51) Int. Cl.: A61B 3/117, A61B 3/135

(54) **Gerät zur Augenuntersuchung mit einer Scheimpflugkamera und einem Spaltprojektor**
Ophthalmic device comprising a Scheimpflug camera and a slit projector
Appareil ophthalmic avec caméra à Scheimpflug et projecteur à fente

(30) Priorität: 04.08.1999 DE 29913602 U
(43) Veröffentlichungstag der Anmeldung: 07.02.2001
(73) Patentinhaber: Oculus Optikgeräte GmbH, 35582 Wetzlar (DE)
(72) Erfinder: Köst, Gert, 30171 Hannover (DE); Repnow, Marc, 28213 Bremen (DE)
(74) Vertreter: Missling, Arne, Dipl.-Ing.

(56) Entgegenhaltungen:
- US-A- 4 171 877
- US-A- 5 886 768
- DRAGOMIRESCU V ET AL: "DEVELOPMENT OF A NEW EQUIPMENT FOR ROTATING SLIT IMAGE PHOTOGRAPHY ACCORDING TO SCHEIMPFLUG'S PRINCIPLE" INTERDISCIPLINARY TOPICS IN GERONTOLOGY,CH,KARGER, BASEL, Bd. 13, 1978, Seiten 118-130, XP000613975 ISSN: 0074-1132

## Beschreibung

Die Erfindung betrifft ein Gerät zur Augenuntersuchung mit einer Scheimpflugkamera und einem Spaltprojektor zur Aufnahme von Schnittbildern eines Auges, welche von einem Ständer getragen ist, wobei der Spaltprojektor und die Scheimpflugkamera um eine gemeinsame Achse drehbar angeordnet sind, welche im Wesentlichen mit der optischen Achse des Auges zusammenfällt.

Geräte zur Augenuntersuchung mit Scheimpflugkameras bieten die Möglichkeit zur Diagnose der vorderen Augenkammer. Im Grunde ermöglichen sie eine besondere Form der Spaltlampenuntersuchung, wobei die beleuchtete Ebene des Auges fotografiert wird. Ein solches Gerät zur Augenuntersuchung mit einer Scheimpflugkamera weist nämlich einen Spaltprojektor auf, wie er auch in herkömmlichen Spaltlampen verwendet werden kann.

Derartige Spaltprojektoren sind aus dem Stand der Technik bekannt. Das Prinzip des Spaltprojektors beruht darauf, dass die brechenden Medien der Augenvorderkammer nicht glasklar sind, sondern an ihnen, insbesondere im kurzwelligen Anteil des sichtbaren Lichtes, eine deutliche Streuung erfolgt. Dies führt dazu, dass ein scharf gebündelter Lichtstrahl, d. h. der projizierte Spalt, den man durch die optischen Medien des Auges schickt, bei seitlicher Betrachtung in ihnen sichtbar wird, ähnlich wie Lichtstrahlen eines Scheinwerfers im Nebel. Die verschiedenen Anteile der brechenden Medien des Auges haben eine unterschiedlich starke Lichtstreuung und können daher unterschieden werden. Das Prinzip dieser fokalen Beleuchtung wird in dem Spaltprojektor perfektioniert. Zur Beleuchtung wird ein spaltförmiges Lichtbündel hoher Lichthelligkeit und möglichst hoher Farbtemperatur verwendet.

Die mittels des Spaltprojektors erleuchtete Ebene in dem Auge kann mittels einer Scheimpflugkamera fotografiert werden. Eine Scheimpflugkamera ist eine Kamera, die der Scheimpflugbedingung genügt. Die Scheimpflugbedingung fordert, dass die Ebene der Dingpunkte, dies ist hier die erleuchtete Ebene in dem Auge, die Hauptebene des Kamera-Linsensystemes und die Bildebene sich in einer gemeinsamen Achse schneiden. Durch das Kippen der Bildebene gegenüber der Hauptebene des Kamera-Linsensystemes, kann die Dingebene in einer beliebigen räumlichen Lage sein, wobei in der Schärfentiefenzone Dingpunkte erfasst werden können, die bei senkrechter Dingebene nicht zugleich scharf abgebildet werden können.

Ein derartiges Gerät ist beispielsweise auch aus der US 4 171 877 bekannt. Der Nachteil des in dieser Druckschrift offenbarten Gerätes liegt darin, dass die Spannungsversorgung und auch die Datenübertragung mittels Kabeln erfolgt. Außerdem sind drehfeste Teile der Scheimpflugkameras so montiert, so dass sie im Drehweg drehbarer Teile der Scheimpflugkamera liegen. Dadurch ist die Drehbarkeit der Scheimpflugkamera und des Spaltprojektors beschränkt. Dieses hat den Nachteil, dass eine relativ aufwendige Steuerung für die Drehbewegung der Scheimpflugkamera und des Spaltprojektors vorgesehen sein muss, so dass z.B. die Kabel für die Spannungsversorgung bzw. die Datenübertragung nicht abgerissen werden. Eine mehrmalige Abtastung des Auges ist somit immer von einer Rückführung der Scheimpflugkamera und des Spaltprojektors in eine Ausgangslage unterbrochen. So kommt es während der Augenuntersuchung immer wieder zu störenden Unterbrechungen, die auf die mechanische Konstruktion der Scheimpflugkamera zurückzuführen sind.

Der Erfindung liegt daher die Aufgabe zugrunde, ein Gerät zur Augenuntersuchung vorzuschlagen, mit dem ohne Unterbrechungen und Rückführungen zu einem Ausgangspunkt das Auge mehrfach abgetastet werden kann.

Diese Aufgabe wird erfindungsgemäß dadurch gelöst, dass der Spaltprojektor und die Scheimpflugkamera um mehr als 360° um die Achse frei drehbar sind, und dass über einen Übertrager sowohl eine Spannungsversorgung für die Scheimpflugkamera und dem Spaltprojektor besteht.

Durch den Übertrager wird eine drahtlose Verbindung zur Spannungsversorgung, der Scheimpflugkamera und/oder dem Spaltprojektor ermöglicht. Der Drehwinkel der Scheimpflugkamera und des Spaltprojektors ist daher nicht mehr durch Kabel beschränkt. Durch mehrfache Umdrehungen des Spaltprojektors und der Scheimpflugkamera kann so das Auge in schneller Folge abgetastet werden und Schnittbilder angefertigt werden.

Gemäß der Erfindung ist der Übertrager ein Schleifringübertrager. Vorteilhaft sind die Scheimpflugkamera und der Spaltprojektor auf einem an dem Ständer drehbar gelagerten Rotor angebracht, wobei die Drehachse des Rotors die optische Achse des Spaltprojektors ist. Gemäß der Erfindung kann dann auf dem Rotor eine Recheneinheit angebracht sein, welche mit der Scheimpflugkamera verbunden ist.

Vorzugsweise kann mit der Scheimpflugkamera und/oder der Recheneinheit eine drahtlose Datenübertragungsstrecke bestehen. Diese drahtlose Datenübertragungsstrecke kann vorteilhaft über den Schleifringübertrager geführt sein. Ebenso wie die Spannungsversorgung mittels eines Übertragers eignet sich die drahtlose Datenübertragungsstrecke dazu, dass die Scheimpflugkamera mit dem Spaltprojektor um beliebige Winkel drehbar ist. Dadurch, dass bereits auf dem Rotor eine Recheneinheit installiert ist, kann dort schon ein Großteil der vorzunehmenden Datenverarbeitung der von der Scheimpflugkamera gelieferten Bilddaten erfolgen. Insbesondere wenn, wie es besonders vorteilhaft ist, eine CCD-Kamera oder C-MOS-Kamera verwendet wird, kann das von diesen Kameras gelieferte digitale Signal der Recheneinheit in ein analoges Signal umgewandelt werden, wodurch nur zwei elektrische Signale über den Übertrager geführt werden müssen, statt der für eine digitale Kodierung üblichen 16 Signale.

Der Ständer einer Scheimpflugkamera kann vorteilhaft in drei Raumachsen verfahren werden.

In einer besonderen Ausführungsform der Scheimpflugkamera hat der Spaltprojektor eine Lichtquelle, eine vor der Lichtquelle angeordnete Spaltblende und ein der Spaltblende nachgeordnetes Linsensystem. Die Lichtquelle besteht dabei aus mehreren nebeneinander und im Wesentlichen in Spaltlängsrichtung, d. h. in der Ebene des projizierten Spaltes, angeordneten Leuchtdioden.

Die vorzugsweise Verwendung von Leuchtdioden als Lichtquelle in der Scheimpflugkamera bringt gleich mehrere Vorteile. Der für den Augenarzt bedeutendste Vorteil liegt in der hohen Geschwindigkeit, mit der die für die Schnittbildfotografie notwendigen Blitze mit den Leuchtdioden erzeugt werden können. Darüber hinaus sind Leuchtdioden wesentlich kompakter als die herkömmlichen Lichtquellen; die Leuchtdioden sind robuster als beispielsweise hochempfindliche Xenon-Hochdrucklampen und spürbar preiswerter.

Gemäß bevorzugter Ausführungsformen können die Leuchtdioden der Scheimpflugkamera in dieser besonderen Ausführungsform bogen- oder kreisförmig angeordnet sein. Die Krümmung des Bogens ist dann vorteilhaft so bestimmt, dass das Linsensystem von einer größtmöglichen Lichtmenge passiert wird. Es ist aber auch möglich, dass in der besonderen Ausführungsform der Scheimpflugkamera die Leuchtdioden im Wesentlichen in einer Ebene parallel zu der Spaltblende angeordnet sind. Vorteilhaft sind dann die Hauptstrahlen der Leuchtdioden relativ zur optischen Achse des Linsensystems geneigt, und die Neigung der Hauptstrahlen entspricht dem Abstand der Leuchtdioden zur optischen Achse. Vorteilhaft schneiden sich die Hauptstrahlen im Wesentlichen in einem gemeinsamen Schnittpunkt.

Der Spaltprojektor, in der besonderen Ausführungsform der erfindungsgemäßen Scheimpflugkamera, kann vorteilhaft vor dem Linsensystem eine zweite Spaltblende aufweisen, wobei der Spalt dieser zweiten Spaltblende mit dem Spalt der ersten Spaltblende gleichachsig ist und fluchtet.

Gemäß bevorzugter Ausführungsformen können die Leuchtfelder der Leuchtdioden aus Leuchtdioden-Chips bestehen. Diese Leuchtdioden-Chips sind dann vorteilhaft entlang einer Geraden angeordnet, wobei die Anschlussfelder beiderseits der Geraden liegen. Durch diese Anordnung der Leuchtdioden-Chips wird erreicht, dass die durch die Anschlussfelder abgeschatteten Bereiche der Leuchtdioden-Chips möglichst am Rande der auf die erste Spaltblende abgebildeten Leuchtfelder liegen und eine größtmögliche Lichtmenge das Linsensystem erreicht.

Gemäß bevorzugter Ausführungsformen können die Diodenlinsen astigmatisch sein und/oder das Linsensystem aus Zylinderlinsen bestehen. In beiden Fällen wird eine Bündelung des von den Leuchtfeldern erzeugten Lichtes auf den Spalt in der ersten Spaltblende erreicht.

Die erste Spaltblende kann vorzugsweise eine Breite von 50 bis 120 um haben, während der Öffnungswinkel des aus dieser ersten Spaltblende austretenden Strahlenbündels nicht größer als 2,9° sein soll. Um ein vorteilhaftes Streuverhalten zu erhalten, werden Leuchtdioden bevorzugt, welche blaues Licht erzeugen.

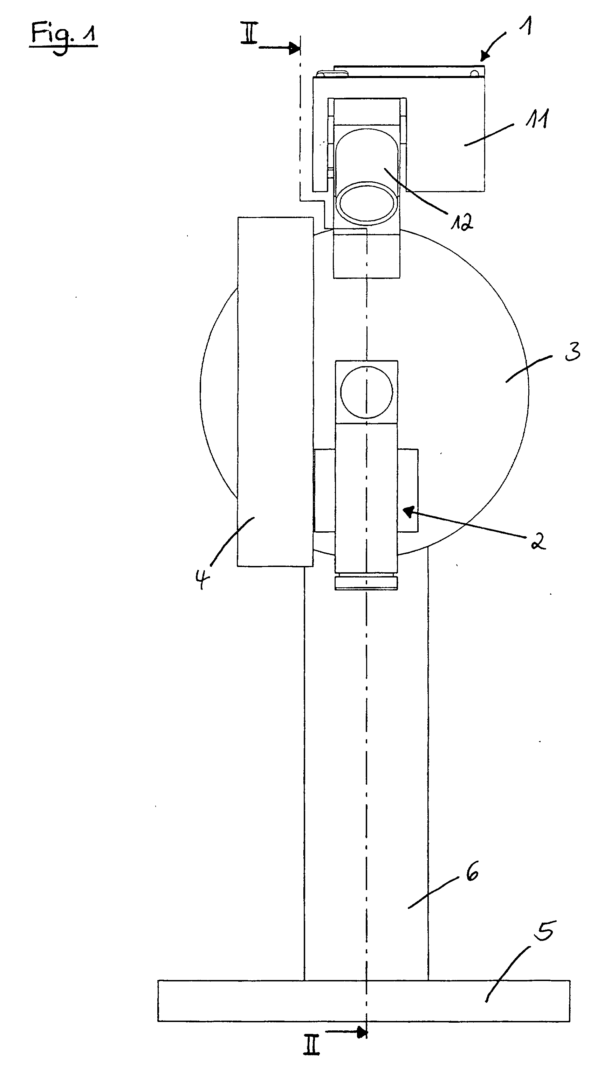

Ein Ausführungsbeispiel ist anhand der Zeichnung näher beschrieben. Darin zeigt
- Fig. 1: eine Vorderansicht eines erfindungsgemäßen Gerätes,

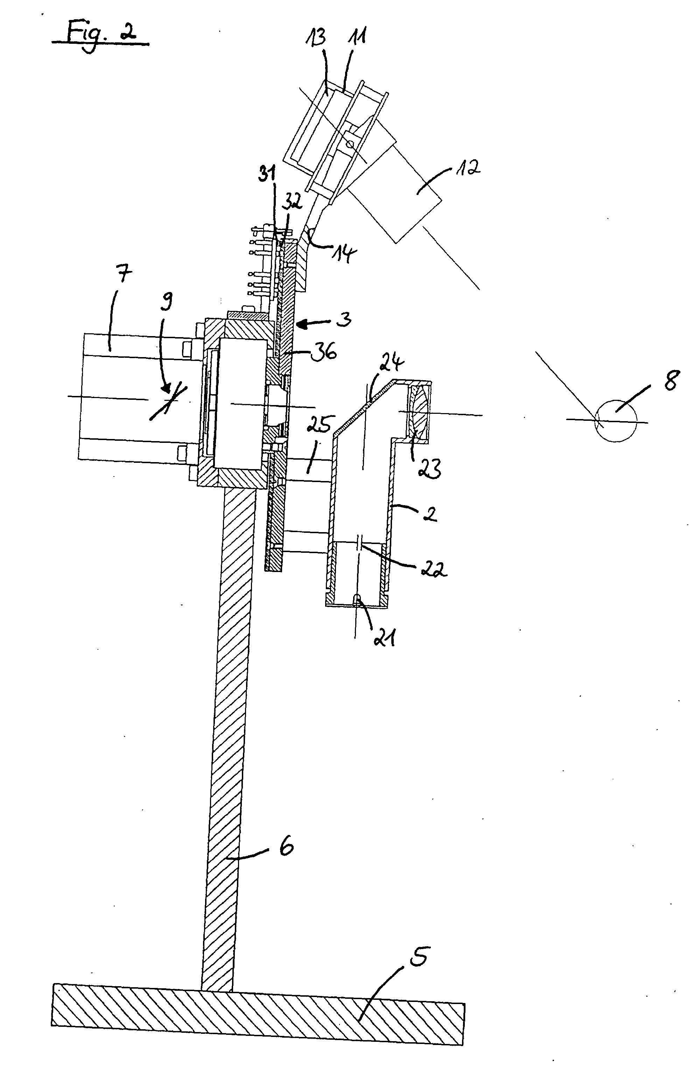

- Fig. 2: einen seitlichen Schnitt durch das Gerät gemäß der Linie II, II in Fig. 1,

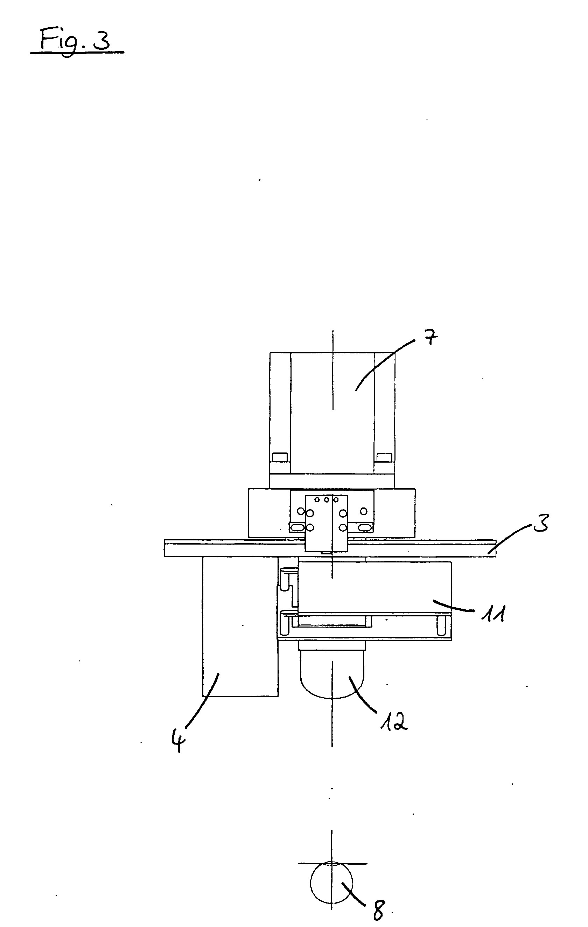

- Fig. 3: eine Draufsicht auf das Gerät gemäß Fig. 1,

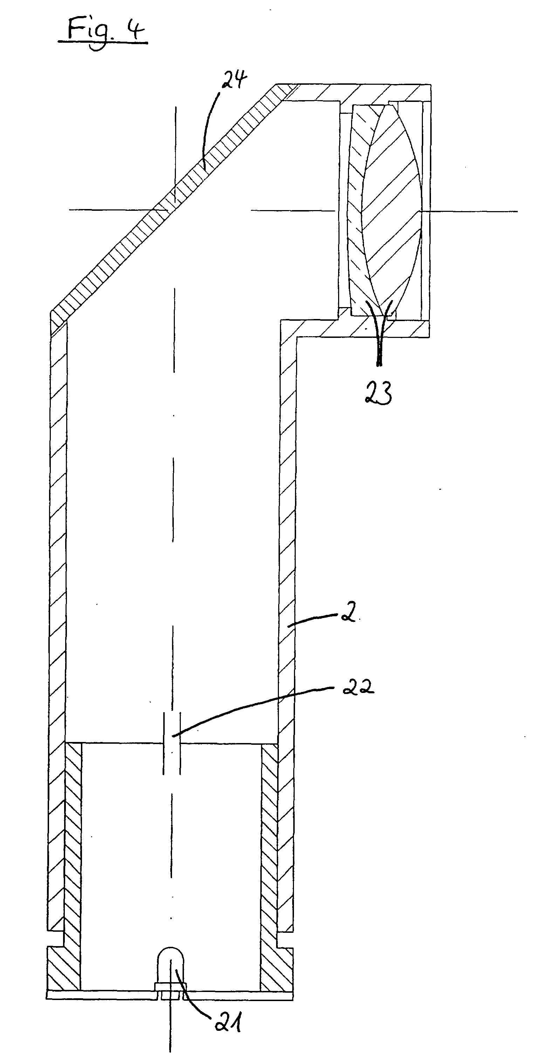

- Fig. 4: den Spaltprojektor einer Scheimpflugkamera gemäß der Fig. 1 bis 3 und

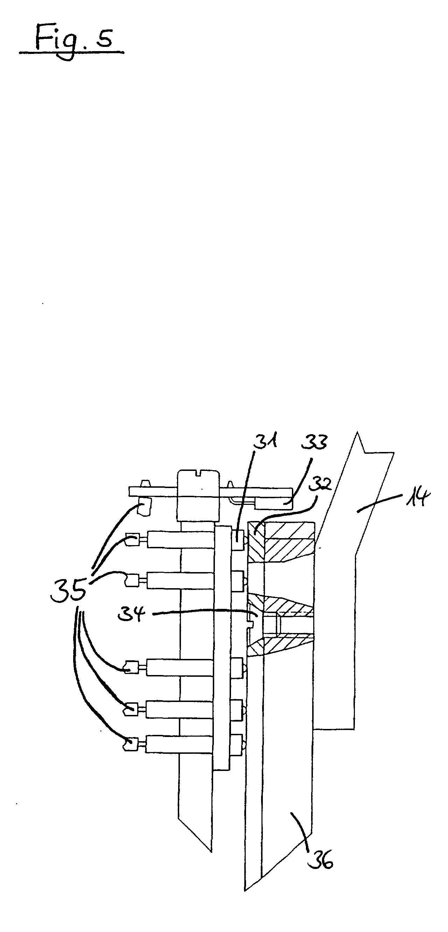

- Fig. 5: eine Einzelheit des Schleifringübertragers des Gerätes gemäß den Fig. 1 bis 3.

Das dargestellte Gerät mit einer Scheimpflugkamera 1 und einem Spaltprojektor 2 weist einen Ständer 6 auf, der auf einem Fuß 5 ruht. An dem Ständer 6 ist ein Rotor 3 drehbar gelagert. Der Rotor 3 ist durch einen Motor 7 antreibbar. Bei dem Motor 7 handelt es sich vorzugsweise um einen Schrittmotor.

An dem Rotor 3 wiederum sind über einem Halter 14 bzw. einem Halter 25 die Scheimpflugkamera 1 und der Spaltprojektor 2 befestigt. Die optische Achse der aus dem Spaltprojektor 2 austretenden Strahlen fällt dabei mit der Drehachse des Rotors zusammen.

Der Spaltprojektor 2 weist als Lichtquelle eine Diodenreihe 21 auf. Diese Diodenreihe 21 beleuchtet eine Spaltblende 22, wodurch die Spaltblende gemäß dem Köhlerschen Abbildungsschema über einem Spiegel 24 in dem Projektor-Linsensystem 23 abgebildet wird. Dort wird das durch den Spalt der Spaltblende 22 tretende Licht gebündelt, um schließlich in einem Auge 8 eines Probanden eine Ebene zu beleuchten. Diese Ebene kann nun mittels der Scheimpflugkamera 1 aufgenommen werden. Dazu weist die Scheimpflugkamera 1 ein Kamera-Linsensystem 12 auf, welches vor dem Gehäuse 11 der Scheimpflugkamera montiert ist. In dem Gehäuse 11 der Scheimpflugkamera 1 ist in der Bildebene 13 ein CCD-Chip angeordnet, mit welchem die Bildaufnahme erfolgt. Um der Scheimpflugbedingung genüge zu tun, sind dabei die Bildebene 13, die Hauptebene des Kamera-Linsensystemes 12 sowie die betrachtete und beleuchtete Ebene in dem Auge 8 des Probanden so zueinander geneigt, dass sie sich in einer gemeinsamen Achse 9 schneiden.

Das von der Scheimpflugkamera aufgenommene Bild wird in der auf dem Rotor 3 montierten Recheneinheit 4 in ein analoges Videosignal gewandelt. Dieses analoge Videosignal wird auf zwei Schleifringe 32 geleitet, welche an dem Rotor 3 mittels Schrauben 34 befestigt sind. An dem Ständer 6 sind weiter Schleifringkontakte 31 befestigt, mit welchen das Videosignal von den Schleifringen 32 abgegriffen wird. Das Videosignal gelangt dann über Anschlussleitungen 35 zu einem nicht dargestellten Rechner und/oder Monitor. Zusätzlich zu den beiden Schleifringübertragern für das Videosignal sind weitere drei Schleifringe 32 bzw. weiter drei Schleifkontakte 31 an dem Rotor 3 bzw. an dem Ständer 6 vorgesehen, mit welchen eine Spannungsversorgung des Spaltprojektors 2 und der Scheimpflugkamera 1 erfolgt. Des Weiteren ist an dem Ständer 6 ein Sensor 33 angebracht, welcher den Drehwinkel des Rotors 3 mit der Scheimpflugkamera 1 und dem Spaltprojektor 2 misst. Somit kann unmittelbar festgestellt werden, in welcher Winkelstellung sich Scheimpflugkamera und Spaltprojektor 2 zu einer Ausgangsposition befinden.

Der Vorteil dieses Ausführungsbeispieles eines erfindungsgemäßen Gerätes ist, dass wegen des Schleifringübertragers beliebige Drehwinkel des Rotors und damit der Scheimpflugkamera und des Spaltprojektors gefahren werden können. Dadurch kann das gesamte Auge abgetastet werden und dies auch, ohne dass die Scheimpflugkamera oder der Spaltprojektor in eine Ausgangsposition zurückgefahren werden müssen. Auch ein mehrfaches Abtasten des Auges ist möglich. Die Abtastung erfolgt wesentlich schneller als bei Geräten nach dem Stand der Technik, da der Spaltprojektor durch seine Ausstattung mit Leuchtdioden als Leuchtquellen, im Gegensatz zu den in dem Stand der Technik üblichen Leuchtquellen, sehr schnelle und helle Blitze zünden kann. Das dargestellte Gerät ist darüber hinaus wesentlich kostengünstiger herzustellen als die im Stand der Technik üblichen Scheimpflugkameras. Durch den Verzicht auf empfindliche Xenon-Hochdrucklampen kann der Spaltprojektor wesentlich kompakter gestaltet werden, da eine zusätzliche Kühleinrichtung entfallen kann.

### Bezugszeichenliste

- 1 -: Scheimpflugkamera
- 11 -: Gehäuse
- 12 -: Objektiv/Kamera-Linsensystem
- 13 -: Bildebene
- 14 -: Kamerahalter

- 2 -: Spaltprojektor
- 21 -: Leuchtdioden
- 22 -: Spaltblende
- 23 -: Projektor-Linsensystem
- 24 -: Spiegel
- 25 -: Projektorhalter

- 3 -: Rotor
- 31 -: Schleifkontakte
- 32 -: Schleifring
- 33 -: Sensor
- 34 -: Schraube
- 35 -: Anschlussleitungen
- 36 -: Rotorscheibe

- 4 -: Gehäuse der Recheneinheit
- 5 -: Fuß
- 6 -: Ständer
- 7 -: Motor
- 8 -: Auge
- 9 -: gemeinsame Schnittachse

## Patentansprüche

1. Gerät zur Augenuntersuchung mit einer Scheimpflugkamera (1) und einem Spaltprojektor (2) zur Aufnahme von Schnittbildern eines Auges (8), welche von einem Ständer (6) getragen ist, wobei der Spaltprojektor (2) und die Scheimpflugkamera (1) um eine gemeinsame Achse drehbar angeordnet sind, welche im Wesentlichen mit der optischen Achse des Auges (8) zusammenfällt,
**dadurch gekennzeichnet,**
**dass** der Spaltprojektor (2) und die Scheimpflugkamera (1) um mehr als 360° um die Achse frei drehbar sind und dass über einen Übertrager sowohl eine Spannungsversorgung für die Scheimpflugkamera (1) und den Spaltprojektor (2) besteht, wobei
der Übertrager ein Schleifringübertrager (31, 32) ist.

2. Gerät nach Anspruch 1, **dadurch gekennzeichnet, dass** die Scheimpflugkamera (1) und der Spaltprojektor (2) auf einem an den Ständer (6) drehbar gelagerten Rotor (3) angebracht sind, dessen Drehachse die optische Achse des Spaltprojektors (2) ist.

3. Gerät nach Anspruch 2, **dadurch gekennzeichnet, dass** auf dem Rotor (3) eine Recheneinheit (4) angebracht ist, die mit der Scheimpflugkamera (1) verbunden ist.

4. Gerät nach einem der Ansprüche 1 bis 3, **dadurch gekennzeichnet, dass** eine drahtlose, mit der Scheimpflugkamera (1), dem Spaltprojektor (2) und/oder der Recheneinheit (4) verbundene Datenübertragungsstrecke besteht.

5. Gerät nach einem der Ansprüche 1 bis 4 **dadurch gekennzeichnet, dass** über den Schleifringübertrager eine Datenübertragungsstrecke mit der Scheimpflugkamera (1), dem Spaltprojektor (2) und/oder der Recheneinheit (4) besteht.

6. Gerät nach einem der Ansprüche 1 bis 5, **dadurch gekennzeichnet, dass** die Scheimpflugkamera (1) eine CCD-Kamera ist.

7. Gerät nach einem der Ansprüche 1 bis 6, **dadurch gekennzeichnet, dass** die Scheimpflugkamera (1) eine C-MOS-Kamera ist.

8. Gerät nach einem der Ansprüche 1 bis 7, **dadurch gekennzeichnet, dass** der Ständer (6) in drei Raumachsen verfahrbar ist.

9. Gerät nach einem der Ansprüche 1 bis 8, wobei der Spaltprojektor (2) eine Lichtquelle, eine vor der Lichtquelle angeordnete Spaltblende (22) und ein der Spaltblende (22) nachgeordnetes Linsensystem (23) aufweist, **dadurch gekennzeichnet, dass** die Lichtquelle aus mehreren nebeneinander und im Wesentlichen in Spaltlängsrichtung, d. h. in der Ebene des projizierten Spaltes angeordneten Leuchtdioden (21) besteht.

10. Gerät nach einem der Ansprüche 1 bis 9, **dadurch gekennzeichnet, dass** sich in dem Strahlengang des Spaltprojektors (2) und/oder der Scheimpflugkamera (1) ein dichroitischer Spiegel (24) befindet.

11. Gerät nach Anspruch 9 oder 10, **dadurch gekennzeichnet, dass** die Leuchtdioden (21) bogen- oder kreisförmig in der Ebene des projizierten Spaltes angeordnet sind.

12. Gerät nach Anspruch 11, **dadurch gekennzeichnet, dass** die Krümmung des Bogens so bestimmt ist, dass das Linsensystem (23) von größtmöglichen Lichtmengen passiert wird.

13. Gerät nach Anspruch 9, **dadurch gekennzeichnet, dass** die Leuchtdioden (21) achsparallel zu der Spaltblende (22) angeordnet sind.

14. Gerät nach Anspruch 13, **dadurch gekennzeichnet, dass** Hauptstrahlen der Leuchtdioden (21) relativ zur optischen Achse des Linsensystems geneigt sind und dass die Neigung der Hauptstrahlen dem Abstand der Leuchtdioden (21) zur optischen Achse entspricht.

15. Gerät nach Anspruch 14, **dadurch gekennzeichnet, dass** die Hauptstrahlen der Leuchtdioden (21) sich im Wesentlichen in einem gemeinsamen Punkt schneiden.

16. Gerät nach einem der Ansprüche 9 bis 15, **dadurch gekennzeichnet, dass** vor dem Linsensystem eine zweite Spaltblende angeordnet ist, wobei der Spalt dieser zweiten Spaltblende mit dem Spalt der ersten Spaltblende (22) fluchtet.

17. Gerät nach einem der Ansprüche 9 bis 16, **dadurch gekennzeichnet, dass** die Leuchtdiode (21) Leuchtdioden-Chips aufweist.

18. Gerät nach Anspruch 17, **dadurch gekennzeichnet, dass** die Leuchtdioden-Chips entlang einer Geraden angeordnet sind, wobei die Anschlussfelder beiderseits der Geraden liegen.

19. Gerät nach einem der Ansprüche 9 bis 18, **dadurch gekennzeichnet, dass** das Linsensystem (23) astigmatisch ist.

20. Gerät nach einem der Ansprüche 9 bis 19, **dadurch gekennzeichnet, dass** das Linsensystem (23) Zylinderlinsen aufweist.

21. Gerät nach einem der Ansprüche 1 bis 20, **dadurch gekennzeichnet, dass** der Spalt der ersten Spaltblende (22) eine Breite von 50 bis 120 µm hat.

22. Gerät nach einem der Ansprüche 1 bis 21, **dadurch gekennzeichnet, dass** der Öffnungswinkel des aus der ersten Spaltblende (22) austretenden Strahlenbündels nicht größer als 2,9° ist.

23. Gerät nach einem der Ansprüche 10 bis 22, **dadurch gekennzeichnet, dass** die Leuchtdioden (21) blaues Licht erzeugen.

## Claims

1. An instrument for examining the eye having a Scheimpflug camera (1) and an aperture projector (2) for recording sectional images of an eye (8), which camera is borne on a stand (6), the aperture projector (2) and the Scheimpflug camera (1) being positioned rotatable about a common axis which substantially corresponds to the optical axis of the eye (8),
**characterised in that** the aperture projector (2) and the Scheimpflug camera (1) are freely rotatable by more than 360° about the axis, and **in that** a voltage supply for both the Scheimpflug camera (1) and the aperture projector (2) is provided by a transformer, the transformer being a slip-ring transformer (31, 32).

2. An instrument according to Claim 1, **characterised in that** the Scheimpflug camera (1) and the aperture projector (2) are fitted on a rotor (3) rotatably mounted on the stand (6), the axis of rotation of the said rotor being the optical axis of the aperture projector (2).

3. An instrument according to Claim 2, **characterised in that** an arithmetic unit (4) is fitted on the rotor (3), which arithmetic unit is connected to the Scheimpflug camera (1).

4. An instrument according to Claim 1 to 3, **characterised in that** a wireless data-transmission line exists, which is connected to the Scheimpflug camera (1), the aperture projector (2) and/or the arithmetic unit (4).

5. An instrument according to one of Claims 1 to 4, **characterised in that** via the slip-ring transformer a data-transmission line is provided by the Scheimpflug camera (1), the aperture projector (2) and/or the arithmetic unit (4).

6. An instrument according to one of Claims 1 to 5, **characterised in that** the Scheimpflug camera (1) is a CCD camera.

7. An instrument according to one of Claims 1 to 6, **characterised in that** the Scheimpflug camera (1) is a C-MOS camera.

8. An instrument according to one of Claims 1 to 7, **characterised in that** the stand (6) is driveable in three spatial axes.

9. An instrument according to one of Claims 1 to 8, wherein the aperture projector (2) has a light source, an aperture slit (22) positioned in front of the light source, and a lens system (23) positioned downstream of the aperture slit (22), **characterised in that** the light source consists of a plurality of light-emitting diodes (21) arranged alongside one another and substantially in the direction of the slit length, i.e. within the plane of the projected slit.

10. An instrument according to one of Claims 1 to 9, **characterised in that** a dichroic mirror (24) is positioned in the beam path of the aperture projector (2) and/or of the Scheimpflug camera (1).

11. An instrument according to Claim 9 or 10, **characterised in that** the light-emitting diodes (21) are arranged in the form of an arc or a circle within the plane of the projected slit.

12. An instrument according to Claim 11, **characterised in that** the curvature of the arc is determined such that as much light as possible passes through the lens system (23).

13. An instrument according to Claim 9, **characterised in that** the light-emitting diodes (21) are arranged axially parallel to the aperture slit (22).

14. An instrument according to Claim 13, **characterised in that** main beams of the light-emitting diodes (21) are inclined relative to the optical axis of the lens system, and **in that** the incline of the main beams corresponds to the distance of the light-emitting diodes (21) from the optical axis.

15. An instrument according to Claim 14, **characterised in that** the main beams of the light-emitting diodes (21) intersect substantially at a common point.

16. An instrument according to one of Claims 9 to 15, **characterised in that** a second aperture slit is positioned in front of the lens system, the slit of this second aperture slit being aligned with the slit of the first aperture slit (22).

17. An instrument according to one of Claims 9 to 16, **characterised in that** the light-emitting diode (21) comprises LED chips.

18. An instrument according to Claim 17, **characterised in that** the LED chips are arranged along a straight line, the conductor lands lying on both sides of the straight line.

19. An instrument according to one of Claims 9 to 18, **characterised in that** the lens system (23) is astigmatic.

20. An instrument according to one of Claims 9 to 19, **characterised in that** the lens system (23) comprises cylindrical lenses.

21. An instrument according to one of Claims 1 to 20, **characterised in that** the slit of the first aperture slit (22) has a width of 50 to 120 µm.

22. An instrument according to one of Claims 1 to 21, **characterised in that** the angle of aperture of the bundle of rays emerging from the first aperture slit (22) is not greater than 2.9°.

23. An instrument according to one of Claims 10 to 22, **characterised in that** the light-emitting diodes (21) generate blue light.

## Revendications

1. Appareil pour l'examen de l'oeil, comportant une caméra de Scheimpflug (1) et un projecteur à fente (2) pour photographier des vues en coupe d'un oeil (8), laquelle est portée par un support (6), le projecteur à fente (2) et la caméra de Scheimpflug (1) étant disposés de manière à pouvoir pivoter autour d'un axe commun, qui coïncide essentiellement avec l'axe optique de l'oeil (8),
**caractérisé en ce que**
le projecteur à fente (2) et la caméra de Scheimpflug (1) peuvent pivoter librement de plus de 360° autour de l'axe et **en ce qu'**il existe, par l'intermédiaire d'un transmetteur, une alimentation en tension aussi bien pour la caméra de Scheimpflug (1) que pour le projecteur à fente (2), le transmetteur étant un transmetteur à bagues collectrices (31, 32).

2. Appareil suivant la revendication 1, **caractérisé en ce que** la caméra de Scheimpflug (1) et le projecteur à fente (2) sont montés sur un rotor (3) monté sur le support (6) de manière à pouvoir pivoter, l'axe du rotor étant l'axe optique du projecteur à fente (2).

3. Appareil suivant la revendication 2, **caractérisé en ce que** sur le rotor (3), est installée une unité de calcul (4) qui est reliée à la caméra de Scheimpflug (1).

4. Appareil suivant l'une des revendications 1 à 3, **caractérisé en ce qu'**il existe une ligne de transmission des données, reliée sans fil à la caméra de Scheimpflug (1), au projecteur à fente (2) et/ou à l'unité de calcul (4).

5. Appareil suivant l'une des revendications 1 à 4, **caractérisé en ce que**, par l'intermédiaire du transmetteur à bagues collectrices, il existe une ligne de transmission des données comportant la caméra de Scheimpflug (1), le projecteur à fente (2) et/ou l'unité de calcul (4).

6. Appareil suivant l'une des revendications 1 à 5, **caractérisé en ce que** la caméra de Scheimpflug (1) est une caméra CCD.

7. Appareil suivant l'une des revendications 1 à 6, **caractérisé en ce que** la caméra de Scheimpflug (1) est une caméra C-MOS.

8. Appareil suivant l'une des revendications 1 à 7, **caractérisé en ce que** le support (6) est mobile selon trois axes de l'espace.

9. Appareil suivant l'une des revendications 1 à 8, le projecteur à fente (2) présentant une source de lumière, un diaphragme à fente (22), disposé en avant de la source de lumière, et un système de lentilles (23), disposé en arrière du diaphragme à fente (22), **caractérisé en ce que** la source de lumière est constituée de plusieurs diodes électroluminescentes (21) placées les unes à côté des autres et disposées essentiellement dans la direction longitudinale de la fente, c'est-à-dire dans le plan de projection de la fente.

10. Appareil suivant l'une des revendications 1 à 9, **caractérisé en ce qu'**un miroir dichroïque (24) se trouve sur le trajet des rayons du projecteur à fente (2) et/ou de la caméra de Scheimpflug (1).

11. Appareil suivant la revendication 9 ou 10, **caractérisé en ce que** les diodes électroluminescentes (21) sont disposées en formant un arc ou un cercle dans le plan de projection de la fente.

12. Appareil suivant la revendication 11, **caractérisé en ce que** la courbure de l'arc est déterminée de telle façon que le système de lentilles (23) soit traversé par des quantités de lumière les plus grandes possible.

13. Appareil suivant la revendication 9, **caractérisé en ce que** les diodes électroluminescentes (21) sont disposées suivant un axe parallèle au diaphragme à fente (22).

14. Appareil suivant la revendication 13, **caractérisé en ce que** les rayons principaux des diodes électroluminescentes (21) sont inclinés par rapport à l'axe optique du système de lentilles et **en ce que** l'inclinaison des rayons principaux correspond à la distance des diodes électroluminescentes (21) par rapport à l'axe optique.

15. Appareil suivant la revendication 14, **caractérisé en ce que** les rayons principaux des diodes électroluminescentes (21) se recoupent essentiellement en un point commun.

16. Appareil suivant l'une des revendications 9 à 15, **caractérisé en ce qu'**en avant du système de lentilles, est disposé un deuxième diaphragme à fente, la fente de ce deuxième diaphragme à fente étant alignée avec la fente du premier diaphragme à fente (22).

17. Appareil suivant l'une des revendications 9 à 16, **caractérisé en ce que** la diode électroluminescente (21) présente des puces de diodes électroluminescentes.

18. Appareil suivant la revendication 17, **caractérisé en ce que** les puces de diodes électroluminescentes sont disposées le long d'une droite, les champs de raccordement étant situés des deux côtés de la droite.

19. Appareil suivant l'une des revendications 9 à 18, **caractérisé en ce que** le système de lentilles (23) est astigmate.

20. Appareil suivant l'une des revendications 9 à 19, **caractérisé en ce que** le système de lentilles (23) présente des lentilles cylindriques.

21. Appareil suivant l'une des revendications 1 à 20, **caractérisé en ce que** la fente du premier diaphragme à fente (22) a une largeur de 50 à 120 µm.

22. Appareil suivant l'une des revendications 1 à 21, **caractérisé en ce que** l'angle d'ouverture du faisceau de rayons sortant du premier diaphragme à fente (22) n'est pas plus grand que 2,9°.

23. Appareil suivant l'une des revendications 10 à 22, **caractérisé en ce que** les diodes électroluminescentes (21) produisent une lumière bleue.
